# EUROPÄISCHE PATENTANMELDUNG

(11) **EP 1 568 396 A1**
(43) Veröffentlichungstag der Anmeldung: **31.08.2005**
(21) Anmeldenummer: 05004169.8
(22) Anmeldetag: 25.02.2005
(51) Int. Cl.: A61N 2/00, A61F 7/08

(54) **Schmiegsames Wärmegerät**

(30) Priorität: 27.02.2004 DE 102004009945
(71) Anmelder: Beurer GmbH & Co., 89077 Ulm (DE)
(72) Erfinder: Gingl, Hans-Georg, 89143 Blaubeuren (DE); Merk, Ernst, 89264 Weissenhorn (DE); Schwär, Georg, 89077 Ulm (DE); Köhler, Ralf, 89129 Langenau (DE); Wanner, Reinhold A., 89420 Höchstädt (DE); Bühler, Marco, 89077 Ulm (DE)
(74) Vertreter: Fleck, Hermann-Joseph

(57) **Zusammenfassung**

Die Erfindung bezieht sich auf ein schmiegsames Wärmegerät, wie Heizdecke, Heizkissen, Wärmeunterbett und dgl. mit mindestens einem an eine elektrische Energieversorgung anschließbaren Heizelement (15), das in einen flexiblen Träger (2) eingebettet ist, und mit einer flächig auf oder in dem Träger (2) verteilten Magnetfelderzeugungsanordnung (30). Die kombinierte Funktion des Wärmegerätes mit Magnetfelderzeugung wird dadurch verbessert, dass das Heizelement (15) derart in dem flexiblen Träger (2) verlegt und darin angeordnet ist, dass an der benutzerseitigen Außenseite des flexiblen Trägers (2) eine durch die Dicke mindestens einer Trägerschicht (11, 12) und/oder den Aufbau des Heizelementes (15) vergleichmäßigte Wärmeverteilung erreicht ist und dass die Magnetfelderzeugungsanordnung (30) in der Weise ausgebildet und in oder auf dem flexiblen Träger (2) angeordnet ist, dass auf der benutzerseitigen Außenseite eine magnetische Flussdichte von mindestens 1 mT herrscht und eine punktuelle Wärmekonzentration durch Elemente der Magnetfelderzeugungsanordnung (30) vermieden ist (Fig. 2).

## Beschreibung

Die Erfindung bezieht sich auf ein schmiegsames Wärmegerät, wie Heizdecke, Heizkissen, Wärmeunterbett und dgl. mit mindestens einem an eine elektrische Energieversorgung anschließbaren Heizelement, das in einen flexiblen Träger eingebettet ist, und mit einer flächig auf oder in dem Träger verteilten Magnetfelderzeugungsanordnung.

Ein schmiegsames Wärmegerät dieser Art ist in der DE 33 36 797 A1 erwähnt, wobei zur Ausbildung und Anordnung eines Heizelementes keine näheren Angaben gemacht sind. Dabei ist eine Magnetfelderzeugungsanordnung aus Streifen mit Magnetpartikeln ist so ausgeführt, dass sich eine spezielle Ausrichtung des Magnetfeldes ergibt. Außer Permanentmagnetmaterialien sind auch Elektromagnete erwähnt. Bei der Ausgestaltung eines schmiegsamen Wärmegerätes mit einer Magnetfelderzeugungsanordnung können besondere Probleme auftreten.

Weitere schmiegsame Wärmegeräte mit einer Magnetfelderzeugungsanordnung zeigen auch die DE 89 04 613 U1 und die DE 93 17 115 U1, wobei zu den einzelnen Maßnahmen, wie die Magnetfelderzeugungsanordnung ausgebildet ist, jedoch keine näheren Angaben gemacht sind.

Die DE 297 19 525 U1 zeigt ein schmiegsames Elektromagnetfeld-Therapiegerät in Form einer Matte oder Hülle mit einem Ansteuergerät zum Erzeugen eines speziellen Elektro-Magnetfelds mit einer Grundfrequenz zwischen 18 MHz und 900 MHz und einer einstellbaren Taktfrequenz zwischen 156 Hz und 2500 Hz mittels einer Magnetfeldspule, die als mäanderfrömig verlegter Leitungsdraht ausgebildet ist. Permanentmagnete und Maßnahmen zur Wärmeerzeugung sind nicht genannt.

Der Erfindung liegt die Aufgabe zugrunde, ein schmiegsames Wärmegerät der eingangs genannten Art bereitzustellen, das vorteilhafte Wirkungen für das Wohlbefinden des Benutzers ergibt.

Diese Aufgabe wird mit den Merkmalen des Anspruches 1 gelöst. Hierbei ist vorgesehen, dass das Heizelement derart in dem flexiblen Träger verlegt und darin angeordnet ist, dass an der benutzerseitigen Außenseite des flexiblen Trägers eine durch die Dicke mindestens einer Trägerschicht und/oder den Aufbau des Heizelementes vergleichmäßigte Wärmeverteilung erreicht ist und dass die Magnetfelderzeugungsanordnung in der Weise ausgebildet und in oder auf dem flexiblen Träger angeordnet ist, dass auf der benutzerseitigen Außenseite eine magnetische Flussdichte von mindestens 1 mT herrscht und eine punktuelle Wärmekonzentration durch Elemente der Magnetfelderzeugungsanordnung vermieden ist.

Mit diesen Maßnahmen wird eine für den Benutzer angenehme Wärmeverteilung in Kombination mit einer effizienten Erzeugung von Magnetfeldern auf der Außenseite und Einwirkung von Magnetfeldern auf den Körper des Benutzers sichergestellt.

Dabei bestehen verschiedene Ausgestaltungsmöglichkeiten darin, dass die Magnetfelderzeugungsanordnung als eine Permanentmagnetanordnung und/oder als Resonanz-Magnetfelderzeugungsanurdnung ausgebildet ist, und weiterhin darin, dass die Permanentmagnetanordnung über die Fläche oder einen Flächenbereich des Wärmegerätes verteilt lokale Permanentmagnetelemente oder verteilte, in ein Trägermaterial eingebettete Magnetpartikel aufweist. Hierbei können die Permanentmagnetelemente gleichmäßig verteilt über das Wärmegerät oder unterschiedlich dicht in Abstimmung auf verschiedene Körperregionen angeordnet sein.

Vorteilhafte Eigenschaften ergeben sich ferner dadurch, dass die Permanentmagnetelemente als Magnetfolien, -streifen, oder -scheiben ausgebildet sind.

Auch in Abstimmung auf die Ausbildung eines jeweiligen Wärmegerätes ergeben sich verschiedene Anbringmöglichkeiten dadurch, dass die Magnetelemente in mindestens einer Schicht des flexiblen Trägers oder eines darauf angebrachten separaten Trägerstückes in Taschen aufgenommen, angeklebt, angenietet, angeklipst, an- oder eingeschweißt, an- oder eingelötet, angenäht, angebunden oder mit eingefasst oder mittels Klettverschlussbefestigung angebracht sind.

Eine für eine gleichmäßige Wärmebehandlung einerseits und möglichst effektive Einwirkung der magnetischen Felder günstige Ausgestaltung andererseits besteht darin, dass die Magnetelemente körpernah auf oder in einer Außenschicht des flexiblen Trägers oder einem darauf angebrachten oder anbringbaren flexiblen Trägerstück angeordnet sind, während das Heizelement auf der körperabgewandten Seite der Außenschicht in einer Polsterschichtanordnung angeordnet ist. Gleichzeitig wird durch diese Ausbildung bei kompakter Ausbildung der Permanentmagnete vermieden, dass die erzeugte Wärme in einzelnen Magnetkörpern lokal zu sehr konzentriert wird und eine punktuelle Überhitzung auftritt.

Eine weitere vorteilhafte Ausgestaltung wird dadurch erhalten, dass die Magnetpartikel in mindestens eine Schicht des flexiblen Trägers und/oder eine als Magnetpartikelträger ausgebildete Ummantelung des Heizelements eingearbeitet sind.

Die Fertigung und ein einfacher, robuster Aufbau werden dadurch begünstigt, dass die Magnetpartikel bei einem Herstellungsprozess in die Spritzmasse der Ummantelung oder die Schmelzmasse eines Kunststoff-Schichtmaterials eingebracht und durch ein angelegtes äußeres Magnetfeld ausgerichtet sind.

Ein vorteilhafter Aufbau wird auch durch die Maßnahmen erhalten, dass die Magnetpartikel in Faserverbundstoffe mindestens einer Schicht oder des Heizelementes eingearbeitet sind.

Ein weiterer Vorteil für die Benutzung wird dadurch erhalten, dass mindestens ein Kennzeichnungsmittel für die Positionierung und/oder den Feldlinienverlauf der Magnetfelderzeugungsanordnung vorhanden ist, das als kompassartige Anzeige und/oder Bedruckung ausgebildet ist.

Die Ausführung des Wärmegerätes mit einer Resonanz-Magnetfelderzeugungsanordnung wird dadurch begünstigt, dass zur elektrischen Versorgung von Resonanzfeldspulen der Resonanz-Magnetfelderzeugungsanordnung mindestens eine Leitungsverbindung der elektrischen Versorgung des mindestens einen Heizelementes genutzt ist oder dass eine separate Versorgung, beispielsweise über das Versorgungsnetz, über Batterie oder Akku vorgesehen ist.

Verschiedene Wirkungsweisen lassen sich dadurch erzielen, dass das erzeugte Resonanzmagnetfeld statisch mit Festfrequenzen oder mittels einer Steuereinrichtung in Intensität und Frequenz zeitabhängig veränderbar erzeugbar ist.

Die Erfindung wird nachfolgend anhand von Ausführungsbeispielen unter Bezugnahme auf die Zeichnungen näher erläutert. Es zeigen:
- Fig. 1: ein schmiegsames Wärmegerät in Form eines Heizkissens in teilweise aufgeschnittener, perspektivischer Darstellung,
- Fig. 2: ein Heizkissen mit eingesetzten Permanentmagnetelementen in perspektivischer Darstellung,
- Fig. 3 und 4: einen Ausschnitt des Heizkissens nach Fig. 2 mit beigefügten Permanentmagnetelementen,
- Fig. 5: ein schmiegsames Wärmegerät mit zwei darauf angebrachten separaten Trägerstücken einer Magnetfelderzeugungsanordnung,
- Fig. 6: ein schmiegsames Wärmegerät mit anderer Anordnung von Trägerstücken und
- Fig. 7: eine Heizkordel mit Magnetpartikelträger.

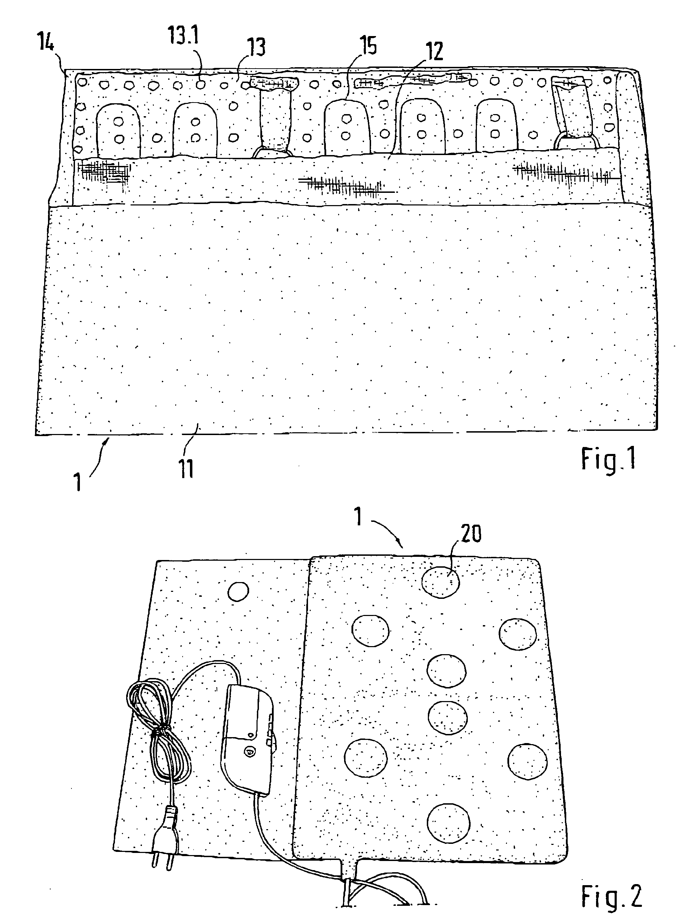

Wie aus Fig. 1 ersichtlich, ist bei dem beispielhaft als Heizkissen ausgebildeten schmiegsamen Wärmegerät 1 ein Aufbau aus mehreren Schichten vorgesehen, nämlich einer ersten Außenschicht 11, einer ersten und zweiten Zwischenschicht 12, 13, zwischen denen ein Heizelement in Form einer Heizkordel 15 mäanderförmig angeordnet und fixiert ist, und einer zweiten Außenschicht 14. Die beiden Außenschichten 11, 14 bestehen vorliegend aus einem leicht zu reinigenden Kunststoffmaterial, während die Zwischenschichten 12, 13 eine Polsterung und auch Beabstandung der Heizkordel 15 ergeben, wodurch auch eine Vergleichmäßigung der abgegebenen Wärme an der Oberfläche des Heizkissens erzielt wird. In einer oder mehreren Schichten bzw. Lagen des Heizkissens ist eine Magnetfelderzeugungsanordnung mit Permanentmagneten und/oder einer Resonanz-Magnetfelderzeugungsanordnung eingebracht. Bei einer Heizdecke oder einem Wärmeunterbett kann auch ein anderer Aufbau und eine andere Anordnung der Schichten bzw. Lagen gewählt sein. Das Heizelement kann auch als Flächenheizelement ausgebildet sein.

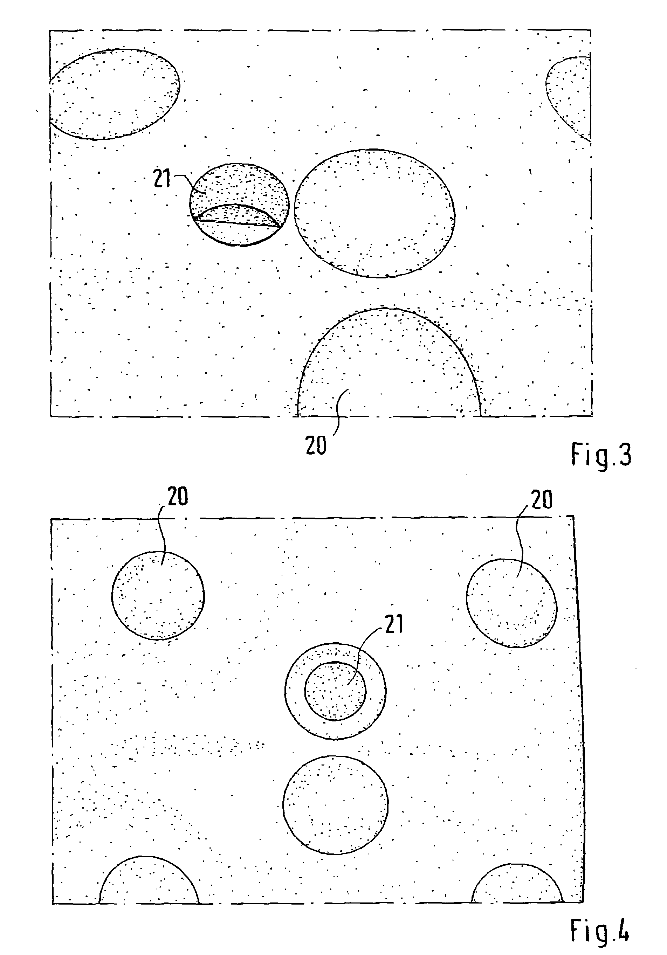

Bei der Ausführung nach Fig. 2 ist in eine der Außenschichten 11, 14, und zwar der dem Benutzer zugekehrten Außenschicht eine Permanentmagnetanordnung 20 eingebracht, bei der Permanentmagnete 21 in in der Außenschicht 11 ausgebildete Magnettaschen 20 eingefügt sind. Die Magnettaschen 20 mit den Magneten 21 sind zumindest über wesentliche Bereiche der Außenschicht 11 verteilt. Die Magnete 21 sind, wie auch die Fig. 3 und 4 zeigen, vorliegend als runde, scheibenförmige Elemente ausgestaltet, die z.B. gummielastisch und/oder schmiegsam beispielsweise als kunststoffgebundene Magnete oder als kompakte Sinter- oder andere Formelemente ausgeführt sind.

Alternativ können auch partikel- bzw. pulverförmige Magnetteilchen in zumindest eine der Schichten 11 bis 14 und/oder die Isolierung der Heizkordel 15 oder z.B. zusammen mit Fixiermaterialien an Fixierstellen 13.1 eingebracht werden.

Nachfolgend werden verschiedene Ausgestaltungsmöglichkeiten im Einzelnen beschrieben. Die Permanentmagnetelemente können z.B. als Magnetfolien, Magnetstreifen oder Magnetscheiben unterschiedlicher Form ausgebildet und durch Kleben, Nieten, Schweißen oder Löten angebracht sein, wobei die Verbindungsart auch in Abhängigkeit von der jeweiligen Trägerschicht optimal gewählt werden kann. In der Trägerschicht, beispielsweise dem Polster, kann für die Magnetelemente 21 auch eine Aussparung angeordnet sein. Die Magnettaschen 20 können ihrerseits in der folienartigen Außenschicht 11 bzw. 14 mittels Schweißen von zwei Folienlagen z.B. durch Hochfrequenzschweißen oder Ultraschallschweißen eingeschweißt sein. Die Taschen können dabei einstückige Magnetelemente oder Magnetpartikel enthalten. Bei mäanderförmig verlegter Heizkordel 15 können die Magnete 21 auch beabstandet von dieser in den Zwischenlagen eingebracht sein, wobei jedoch eine zu große Entfernung vom Körper wegen der dann abgeschwächten magnetischen Wirkung vermieden werden soll. Die Magnete 21 können auch in einem gesonderten Heizkissenbezug mit den eingearbeiteten Magneten 21 angeordnet sein. Auch ein Verkletten von Magnetelementen 21 ist geeignet, wobei z.B. folienartige Magnete variabel positioniert werden können.

Aufgrund einer in Abhängigkeit von dem Aufbau der Magnetelemente 21 möglichen Wärmespeicherwirkung derselben ist es vorteilhaft, die Anordnung der Magnete räumlich von den Heizelementen zu trennen, um eine lokale Überhitzung zu vermeiden. Diese Trennung ergibt sich vorteilhaft dadurch, dass die Magnetelemente 21 mehr an der Oberseite des Heizgerätes und das Heizelement 15 tiefer eingebettet angeordnet ist, da sich dadurch in Körpernähe einerseits eine wirksame magnetische Feldstärke ergibt und andererseits an der Oberfläche eine Vergleichmäßigung der Wärme erhalten wird.

Alternativ oder zusätzlich zu kompakten zusammenhängenden Magnetelementen besteht die Möglichkeit, diese partikelförmig in die Schichten 11 bis 14 des Heizgerätes oder unmittelbar in eine Isolierung des Heizelementes bereits beim Herstellprozess oder auch nachträglich einzuarbeiten, wobei die Partikel in der Größenordnung von Nanometern bis Zentimetern liegen. Dabei ist auf die Polausrichtung der Magnetpartikel zu achten. Hierbei ergibt sich als Vorteil eine flächige Anordnung der Magnetteilchen, die eine gleichmäßige Oberflächentemperatur ohne lokale Wärmespeicherung erreichen lassen, wobei sich eine Wärmebehandlung bzw. Magnetfeldbehandlung über die gesamte oder Teilbereiche der Oberfläche erreichen lässt.

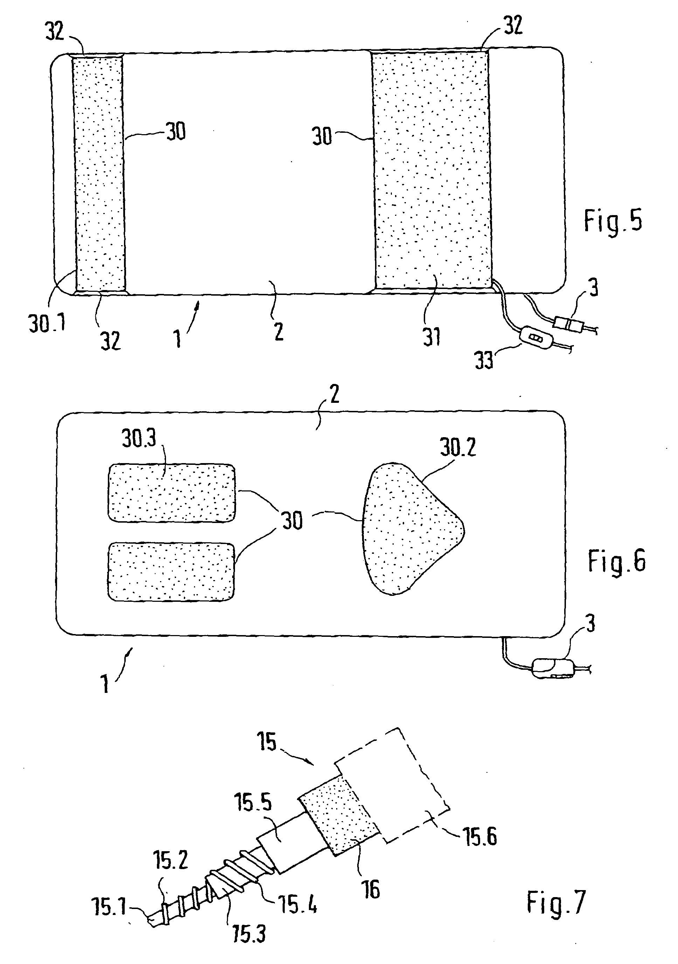

Ein derartiges Ausführunsgbeispiel zeigt Fig. 7. Dabei werden in die Spritzmasse zur Herstellung der Heizkordelummantelung in einen Magnetpartikelträger 16 Magnetpartikel eingeschmolzen, die beim Extrudierprozess nach dem Aufschmelzen durch ein von außen angelegtes Magnetfeld ausgerichtet werden. Nach Fig. 7 beinhaltet das als Heizkordel ausgebildete Heizelement zwei koaxial gewickelte Heizdrähte 15.2 und 15.4, wobei der innere auf einen zentralen Wickelträger 15.1 aus Trägerfäden und der zweite auf einer beide Heizdrähte 15.2 und 15.4 trennenden und bei übermäßiger, insbesondere auch punktueller Erwärmung, schmelzenden Zwischenisolationsschicht 15.3 aufgewickelt ist. Die Zwischenisolationsschicht 15.3 besitzt z.B. NTC-Widerstandsverhalten und ist Teil einer Sicherheitsschaltung, die bei Überschreiten einer gefährdenden Schwellentemperatur bzw. bei Kurzschluss die Wärmevorrichtung abschaltet. In Verbindung mit der Magnetfelderzeugungsanordnung, durch die zusätzliche unerwünschte lokale Erwärmungen hervorgerufen werden können, etwa bei Verwendung kompakter Permanentmagnete, bietet eine solche Sicherheitsschaltung einen zusätzlichen Vorteil. Nach Fig. 7 ist der zweite Heizdraht 15.4 nach außen von einer weiteren Isolierschicht 15.5 umgeben, auf der als weitere Ummantelung der Magnetpartikelträger 16 aufgebracht ist. Auf dieser kann eine Außenisolation 15,6 aufgebracht sein.

Auch können die Magnetpartikel beim Herstellprozess der Polsterung, beispielsweise Schaumlagen, in vliesartige Schichten oder in Kunststofffolien oder Decklagenstoffe oder andere Komponenten eingearbeitet werden, die auch farblich gestaltet sein können, um die Ausrüstung der Geräteoberfläche mit den Permanentmagneten 21 aufzuzeigen.

Als typische Größe kann die magnetische Flussdichte an der dem Benutzer zugekehrten Außenseite bei punktuellen Magnetfeldern z.B. auf 20 bis 40 mT festgelegt werden, die sich im Einzelfall jedoch um ein Mehrfaches nach unten oder oben ändern kann.

Vorteilhaft ist die Ausbildung und Anordnung der Magnetfelderzeugungsvorrichtung in dem flexiblen Träger 2 oder einem darauf angebrachten separaten flexiblen Trägerstück 30.1, 30.2, 30.3 (vgl. Fig. 5 und 6) derart vorgenommen, dass sich an der dem Benutzer zugekehrten Außenseite eine magnetische Flussdichte von mindestens 5 oder 10 mT ergibt.

Vorteilhaft ist auch, zur Anzeige, dass ein Magnetfeld vorhanden und wie es ausgerichtet ist, z.B. eine Art Kompass an dem Heizgerät anzuordnen, der den durch die Magnete 21 erhaltenen Feldlinienverlauf anzeigt. Auch können Kennzeichnungsmittel der Position eingebrachter Magnete 21, beispielsweise eine Bedruckung vorgesehen sein.

Eine weitere Möglichkeit, Magnetteilchen in das Wärmegerät einzuarbeiten, besteht darin, diese unter Polausrichtung in ein Multifilament-Garn einzubringen, das beispielsweise als Trägermaterial eines Heizleiters eingesetzt wird. Bei einer weiteren Ausgestaltung werden Magnetteilchen unter Polausrichtung in die elektrisch leitenden oder nichtleitenden Isolierstoffe, die z.B. ein NTC-Widerstandsverhalten aufweisen, eingearbeitet. Ferner ist es möglich, Magnetteilchen in leitende oder nichtleitende Lacke und Beschichtungen einzuarbeiten, die beispielsweise als Flächenheizelemente oder Isolierungen dienen. Die genannten Maßnahmen sind einzeln vorgesehen oder in im jeweiligen Anwendungsfall in vorteilhafter Kombination miteinander.

Die Magnetpartikel können auch beim Schäumungsprozess eingearbeitet und in ihrer Polung ausgerichtet werden. Ferner besteht eine von der Herstellung abhängige Ausgestaltung darin, dass die Magnetpartikel bei einem Spinprozess von Vliesstoffen oder in gewirkte Materialien sowie in Schichtverbunde, wie z.B. Steppmaterial oder in Polstermaterialien und die Beschichtung von Poistermaterialien eingearbeitet werden. Außerdem können die Magnetpartikel in Folien- und Schutzbezüge beispielsweise beim Herstellen von PVC- oder anderen Schutzfolienmaterialien eingearbeitet werden sowie beim Beschichten, wobei ebenfalls eine Polausrichtung vorgenommen werden kann. Eine weitere Möglichkeit, Magnetpartikel einzubringen, besteht darin, dass diese in Folien zur Fixation der Heizelemente eingearbeitet werden.

Die Polausrichtung der Magnetpartikel im Zusammenhang mit den verschiedenen Materialien kann durch eine beim Herstellprozess festgelegte Materialfließrichtung und Polausrichtung der Magnetteilchen bewirkt werden, wobei auch eine dreidimensionale Formgebung der Magnetteile bewirkt werden kann. Für die Polausrichtung können Siebe mit oder ohne Engstelle im Materialfluss verwendet werden oder starke elektromagnetische Felder um den Materialfluss angelegt werden. Die Formgebung und Größe der Magnetteilchen richtet sich z.B. nach den eingesetzten Fasern von Faserverbundwerkstoffen. Denkbar sind z.B. auch Magnete oder Magnetteilchen in Tropfen- oder Zapfenform.

Weitere Ausführungsbeispiele zur Ausgestaltung eines flexiblen Wärmegerätes 1 mit einer Magnetfelderzeugungsanordnung 30 zeigen die Fig. 5 und 6. Hierbei ist auf dem flexiblen Träger 2, beispielsweise einer Wärmedecke oder einem Wärmeunterbett, an wählbarer Stelle mindestens ein separates Trägerstück mit Magnetelementen aufgebracht, die ihrerseits als Magnetpartikel, kompakten Magneten oder aber als Elektromagnete ausgeführt und z.B. entsprechend vorstehender Beschreibung eingebracht sein können.

Nach Fig. 5 sind zwei unterschiedlich breite, quer verlaufende Trägerstücke 30.1 bzw. 31 auf dem flexiblen Träger 2 aufgebracht, wobei die Verbindung zwischen den separaten Trägerstücken und dem flexiblen Träger 2 entlang einem Abschnitt der Längsseite des flexiblen Trägers 2 mittels dort angeordneter Befestigungsabschnitte 32 vorgenommen ist. Die Befestigungsabschnitte 32 können dabei z.B. miteinander zusammenwirkende Haken- und Ösenelemente sein, wobei die Haken vorteilhaft an den separaten Trägerstücken und die Ösen an dem flexiblen Träger 2 angeordnet sind, oder Klettverbinder mit einerseits an dem flexiblen Träger 2 und andererseits an den Trägerstücken angeordneten Verbindungselementen. Der Elektromagnetträger 31 weist eine eigene elektrische Anschlusseinheit 33 für seine Versorgung und gegebenenfalls variable wählbare Ansteuerung auf und kann somit unabhängig von der Wärmefunktion zugeschaltet, abgeschaltet oder reguliert werden. Das Heizelement 15 ist über einen Versorgungsanschluss 3 mit einer Stromquelle verbunden.

Fig. 6 zeigt eine andere Anordnung von separaten Trägerstücken 30.2, 30.3 auf dem flexiblen Träger 2. Die Befestigung kann beispielsweise mittels einer Klettverbindung oder Hakenverbindung vorgenommen werden. Beispielsweise sind im Beinbereich zwei nebeneinander angeordnete Längsstücke 30.3 aufgebracht, während ein weiteres Trägerstück 30.2 beispielsweise im Schulter- und Nackenbereich angeordnet ist.

Ferner ist als Alternative oder ergänzend vorgesehen, dass die Magnetfelderzeugungsanordnung zum Ausbilden eines Resonanzmagnetfeldes insbesondere mittels Resonanzspulen ausgestaltet ist. Bei Anordnung von Resonanzfeldspulen ist es möglich, Versorgungsleitungen des oder der Heizelemente für die Resonanzfeldspulen zu nutzen, so dass keine zusätzlichen Leitungen bzw. Adern erforderlich sind. Im einfachsten Fall werden nur zwei Leitungsadern zwischen dem Netzstecker und der dazwischen liegenden Schalt- und Kontrolleinheit sowie von dieser zum eigentlichen Heizkörper benötigt. Die Schalt- und Kontrolleinheit kann dabei auch mit einer Schalt- und/oder Kontrollfunktion für die Resonanzfelderzeugung bzw. eine entsprechende Therapiefunktion ausgestattet sein. Bei der Steuerung lassen sich Timerfunktionen verwirklichen, wobei Wärme- und Resonanzfeldtherapie-Funktionen in Abhängigkeit voneinander und von anderen Parametern auch getrennt voneinander eingestellt und gesteuert werden können. Bei einer Ausgestaltung sind dabei auch optische und/oder akustische Anzeigefunktionen für die verschiedenen Betriebsweisen vorgesehen.

Das mittels Resonanzfeldspulen erzeugte Resonanzmagnetfeld wird durch einen Schwingkreis oder Oszillator angeregt und benötigt eine Stromversorgung, die z.B. mittels Akkus, Batterien oder aus dem Leitungsnetz bereitgestellt wird. Bei einer Versorgung über ein Anschlusskabel besteht dieses im einfachsten Fall aus einer zweiadrigen Leitung. Eine zwischengeschaltete Steckkupplung erlaubt die Trennung der Schalt- oder Kontrolleinheit von dem schmiegsamen Teil, so dass z.B. eine Waschmaschinenreinigung ermöglicht wird.

Das Resonanzmagnetfeld kann im einfachsten Fall statisch mit Festfrequenzen oder für eine Therapie in Intensität und Frequenz zeitabhängig veränderbar mit einer entsprechenden Steuervorrichtung erzeugt werden.

## Patentansprüche

1. Schmiegsames Wärmegerät, wie Heizdecke, Heizkissen, Wärmeunterbett und dgl. mit mindestens einem an eine elektrische Energieversorgung anschließbaren Heizelement (15), das in einen flexiblen Träger (2) eingebettet ist, und mit einer flächig auf oder in dem Träger (2) verteilten Magnetfelderzeugungsanordnung (30),
**dadurch gekennzeichnet,**
**dass** das Heizelement (15) derart in dem flexiblen Träger (2) verlegt und darin angeordnet ist, dass an der benutzerseitigen Außenseite des flexiblen Trägers (2) eine durch die Dicke mindestens einer Trägerschicht (11, 12) und/oder den Aufbau des Heizelementes (15) vergleichmäßigte Wärmeverteilung erreicht ist und
**dass** die Magnetfelderzeugungsanordnung (30) in der Weise ausgebildet und in oder auf dem flexiblen Träger (2) angeordnet ist, dass auf der benutzerseitigen Außenseite eine magnetische Flussdichte von mindestens 1 mT herrscht und eine punktuelle Wärmekonzentration durch Elemente der Magnetfelderzeugungsanordnung (30) vermieden ist.

2. Wärmegerät nach Anspruch 1,
**dadurch gekennzeichnet,**
**dass** die Magnetfelderzeugungsanordnung (30) als eine Permanentmagnetanordnung und/oder als Resonanz-Magnetfelderzeugungsanordnung (31) ausgebildet ist.

3. Wärmegerät nach Anspruch 2,
**dadurch gekennzeichnet,**
**dass** die Permanentmagnetanordnung über die Fläche oder einen Flächenbereich des Wärmegerätes verteilt lokale Permanentmagnetelemente (21) oder in ein Trägermaterial verteilt eingebettete Magnetpartikel aufweist.

4. Wärmegerät nach Anspruch 3,
**dadurch gekennzeichnet,**
**dass** die Permanentmagnetelemente (21) als Magnetfolien, -streifen, oder -scheiben ausgebildet sind.

5. Wärmegerät nach Anspruch 3 oder 4,
**dadurch gekennzeichnet,**
**dass** die Magnetelemente (21) in mindestens einer Schicht des flexiblen Trägers (2) oder eines darauf angebrachten flexiblen separaten Trägerstückes (30.1, 30.2, 30.3) in Taschen aufgenommen, angeklebt, angenietet, angeklipst, an- oder eingeschweißt, an- oder eingelötet, angenäht, angebunden oder mit eingefasst oder mittels Klettverschlussbefestigung angebracht sind.

6. Wärmegerät nach einem der Ansprüche 3, 4 oder 5,
**dadurch gekennzeichnet,**
**dass** die Magnetelemente (21) körpernah auf oder in einer Außenschicht (11) des flexiblen Trägers (2) oder einem darauf angebrachten oder anbringbaren flexiblen Trägerstück (30.1, 30.2, 30.3) angeordnet sind, während das Heizelement (15) auf der körperabgewandten Seite der Außenschicht (11) in einer Polsterschichtanordnung (12, 13) angeordnet ist.

7. Wärmegerät nach einem der Ansprüche 3 bis 6,
**dadurch gekennzeichnet,**
**dass** die Magnetpartikel in mindestens eine Schicht (11, 12, 13, 14) des flexiblen Trägers (2) und/oder eine als Magnetpartikelträger (16) ausgebildete Ummantelung des Heizelements (15) eingearbeitet sind.

8. Wärmegerät nach Anspruch 7,
**dadurch gekennzeichnet,**
**dass** die Magnetpartikel bei einem Herstellungsprozess in die Spritzmasse der Ummantelung oder die Schmelzmasse eines Kunststoff-Schichtmaterials eingebracht und durch ein angelegtes äußeres Magnetfeld ausgerichtet sind.

9. Wärmegerät nach Anspruch 7 oder 8,
**dadurch gekennzeichnet,**
**dass** die Magnetpartikel in Faserverbundstoffe mindestens einer Schicht (11, 12, 13, 14) oder des Heizelementes (15) eingearbeitet sind.

10. Wärmegerät nach einem der vorhergehenden Ansprüche,
**dadurch gekennzeichnet,**
**dass** mindestens ein Kennzeichnungsmittel für die Positionierung und/oder den Feldlinienverlauf der Magnetfelderzeugungsanordnung vorhanden ist, das als kompassartige Anzeige und/oder Bedruckung ausgebildet ist.

11. Wärmegerät nach einem der Ansprüche 2 bis 10,
**dadurch gekennzeichnet,**
**dass** zur elektrischen Versorgung von Resonanzfeldspulen der Resonanz-Magnetfelderzeugungsanordnung (31) mindestens eine Leitungsverbindung der elektrischen Versorgung des mindestens einen Heizelementes (15) genutzt ist.

12. Wärmegerät nch Anspruch 11,
**dadurch gekennzeichnet,**
**dass** das erzeugte Resonanzmagnetfeld statisch mit Festfrequenzen oder mittels einer Steuereinrichtung in Intensität und Frequenz zeitabhängig veränderbar erzeugbar ist.

13. Wärmegerät nach einem der vorhergehenden Ansprüche,
**dadurch gekennzeichnet,**
**dass** die Magnetfelderzeugungsanordnung (30) in zumindest einem auf dem flexiblen Träger (2) angebrachten oder anbringbaren Trägerstück (30.1, 30.2, 30.3, 31) aufgenommen ist.
